# EUROPÄISCHE PATENTSCHRIFT

(11) **EP 2 822 567 B1**
(45) Veröffentlichungstag und Bekanntmachung des Hinweises auf die Patenterteilung: **26.08.2020**
(21) Anmeldenummer: 13707872.1
(22) Anmeldetag: 06.03.2013
(51) Int. Cl.: A61K 36/06, A61K 36/064, A61P 37/08

(54) **HEFEEXTRAKT ZUR BEHANDLUNG VON ALLERGIEN**
YEAST EXTRACT FOR TREATING ALLERGIES
EXTRAIT DE LA LEVURE POUR LE TRAITEMENT DES ALLERGIES

(30) Priorität: 06.03.2012 DE 102012101864
(43) Veröffentlichungstag der Anmeldung: 14.01.2015
(73) Patentinhaber: Gramme-Revit GmbH, 99428 Niederzimmern (DE)
(72) Erfinder: LEIBOLD, Wolfgang, 30559 Hannover (DE); DUSEK, Niels, 99428 Niederzimmern (DE)
(74) Vertreter: Kröncke, Rolf
(86) Internationale Anmeldenummer: PCT/EP2013/054483
(87) Internationale Veröffentlichungsnummer: WO 2013/131947

(56) Entgegenhaltungen:
- WO-A2-2004/112776
- WO-A2-2007/135505
- WO-A2-2009/094717
- US-A1- 2006 173 071
- US-A1- 2009 196 921
- R GAUDIBERT: "Les urticaires et oedèmes de Quincke aux moisissures atmosphériques", REVUE FRANCAISE D&'APOS;ALLERGOLOGIE, Bd. 12, Nr. 1, 1972, Seiten 21-34, XP055062783, ISSN: 0035-2845, DOI: 10.1016/S0035-2845(72)80040-4
- MORAES P S A ET AL: "Candida albicans allergen immunotherapy in recurrent vaginal candidiasis", JOURNAL OF INVESTIGATIONAL ALLERGOLOGY AND CLINICAL IMMUNOLOGY, BARCELONA, ES, Bd. 10, Nr. 5, September 2000 (2000-09), Seiten 305-309, XP009169586, ISSN: 1018-9068
- GUSTAFSSON L ET AL: "A CONTROLLED TRIAL OF A TWO-COMPONENT ACELLULAR, A FIVE-COMPONENT ACELLULAR, AND A WHOLE-CELL PERTUSSIS VACCINE", NEW ENGLAND JOURNAL OF MEDICINE, THE, MASSACHUSETTS MEDICAL SOCIETY, WALTHAM, MA, US, Bd. 334, Nr. 6, 8. Februar 1996 (1996-02-08), Seiten 349-355, XP000982706, ISSN: 0028-4793, DOI: 10.1056/NEJM199602083340602
- LEVY D A ET AL: "Phase II study of D.651, an oral vaccine designed to prevent recurrences of vulvovaginal candidiasis", VACCINE, ELSEVIER LTD, GB, Bd. 7, Nr. 4, August 1989 (1989-08), Seiten 337-340, XP023711689, ISSN: 0264-410X, DOI: 10.1016/0264-410X(89)90197-7 [gefunden am 1989-08-01]
- WESTPHAL H J ET AL: "Specific desensitization of patients with chronic urticaria induced by yeasts and with colonization of the intestine by yeasts", DERMATOLOGISCHE MONATSSCHRIFT, JOHANN AMBRISIUS BARTZ, LEIPZIG, DE, DE, Bd. 162, Nr. 11, 1976, Seiten 912-915, XP009169602, ISSN: 0011-9083
- SUNADA YOSUKE ET AL: "Effects of the combined use of Lactobacillus acidophilus strain L-55 and Saccharomyces cerevisiae strain S4-A on experimental allergic rhinitis in BALB/c mice", JOURNAL OF PHARMACOLOGICAL SCIENCES, JAPANESE PHARMACOLOGICAL SOCIETY, TOKYO, JP, Bd. 109, Nr. Suppl. 1, 2009, Seite 79P, XP009169613, ISSN: 1347-8613
- YUMI SEKEKIMURA ET AL: "Anti-allergic effects of a mixture of Saccharomyces cerevisiae and its specific goat's milk immunoglobulin G rich fraction on ovalbumin sensitized BALB/c mice", MILCHWISSENSCHAFT, Bd. 66, Nr. 1, 2011, Seiten 7-10, XP055063224, ISSN: 0026-3788
- SHERRIL L. GREEN ET AL: "ACUTE DIFFUSE MYCOTIC PNEUMONIA IN A 7-MONTH-OLD COLT", VETERINARY RADIOLOGY, Bd. 28, Nr. 6, November 1987 (1987-11), Seiten 216-219, XP55075956, ISSN: 0196-3627, DOI: 10.1111/j.1740-8261.1987.tb00057.x
- K. A. CHAMEROY ET AL: "Effects of a supplement containing chromium and magnesium on morphometric measurements, resting glucose, insulin concentrations and insulin sensitivity in laminitic obese horses", EQUINE VETERINARY JOURNAL, Bd. 43, Nr. 4, 29. September 2010 (2010-09-29), Seiten 494-499, XP055075967, ISSN: 0425-1644, DOI: 10.1111/j.2042-3306.2010.00302.x

## Beschreibung

Die vorliegende Anmeldung betrifft eine Allergie modulierende Zusammensetzung, insbesondere ein Extrakt aus Mikroorganismen, wobei diese Allergie modulierende Zusammensetzung zur mukosalen Verabreichung hergerichtet und ggf. konfektioniert ist wobei diese Allergie modulierende Zusammensetzung innerhalb von maximal zwei Stunden nach Ende des Aussetzens gegenüber dem Allergen verabreicht wird. In einem weiteren Aspekt richtet sich die Anmeldung auf Extrakte aus Mikroorganismen, insbesondere Extrakte aus Mikroorganismen, wie Hefen, erhalten durch Extraktion mit ionischen und nichtionischen Tensidlösungen zur Verwendung bei der Behandlung von Allergien, Autoimmunerkrankungen, Infektionserkrankungen, Hufrehe oder als Adjuvans in Vaccinen.

### Stand der Technik

Die Behandlung von Allergien spielt in der heutigen Zeit eine immer größere Rolle. Tatsächlich nehmen die Befunde von Allergien bei Menschen immer stärker zu. Therapien zur Behandlung von Allergien treten damit immer stärker in den Fokus. Es gibt derzeit unterschiedlichste Verfahren und Methoden eine Allergie zu behandeln. Übliche Verfahren schließen eine Hyposensibilisierung bzw. Desensibilisierung bzw. spezifische Immuntherapie mit oft fraglichem Heilungserfolg ein. Dabei werden den Individuen steigende Mengen an Allergen verabreicht, üblicherweise systemisch. Durch die steigende Menge an Allergen soll eine Desensibilisierung erfolgen. Diese Verfahren dauern üblicherweise Monate bis Jahre und schließen einen häufigen Besuch beim Arzt ein. Weiterhin gibt es Medikamente zur symptomatischen Behandlungen (Corticosteroide, Antihistaminika oder neuere Immunsuppressiva) ohne Heilung, d. h. es findet lediglich eine Behandlung der Symptome statt und keine Behandlung der Ursache.

Es besteht daher nach wie vor ein großer Bedarf geeignete Behandlungen und Therapiekonzepte gegen Allergie bei Mensch und Tier bereitzustellen.

### Beschreibung der Erfindung

Vorliegend werden neue Verwendungen von Mitteln zur Behandlung von Allergien, nämlich von Allergien modulierenden Zusammensetzungen, bereitgestellt, um in der Therapie von Allergien in Individuen eingesetzt zu werden.

Es wurde festgestellt, dass bei der Behandlung der Allergien ein strikter Zeitplan einzuhalten ist, um die Allergien erfolgreich zu behandeln. D. h., es wurde festgestellt, dass das Individuum nach einem Aussetzen gegenüber einem Allergen den folgenden Schritten unterworfen werden sollte:
Nach Beenden des natürlichen oder gezielten Aussetzens gegenüber dem Allergen (in natürlicher oder aufbereiteter Form) wird das Individuum möglichst bald, insbesondere innerhalb von maximal sechs Stunden, wie maximal vier Stunden, insbesondere maximal zwei Stunden nach dem Ende des Aussetzens gegenüber dem Allergen mit einer Dosis der Allergie modulierenden Zusammensetzung behandelt. Dabei ist diese Allergie modulierende Zusammensetzung derart hergerichtet, dass sie zur mukosalen Verabreichung geeignet ist. D. h., die Zusammensetzung wird mukosal, bevorzugt intranasal oder oral, verabreicht. Nach Verabreichen des Mittels zur Behandlung der Allergie, d. h. der Allergie modulierenden Zusammensetzung, wird das Individuum bevorzugt einer möglichst strikten Allergenkarenz insbesondere mindestens 36 Stunden bis 92 Stunden ausgesetzt. In diesem Zusammenhang bedeutet der Ausdruck Allergenkarenz, dass das Individuum möglichst konsequent dem Allergen nicht ausgesetzt wird, das seine zu behandelnde Allergie auslösen kann. Bevorzugt wird deshalb das Individuum nach exogener Allergenexposition vor oder unmittelbar nach Verabreichung des Mittels, gründlich gesäubert (Duschen, Kleiderwechsel, Tiere werden geduscht und trocken gerieben oder feucht abgewaschen), um mögliche Allergenspuren an Haut, Haaren bzw. Kleidung sicher zu entfernen.

Nach Ablauf des Karenzzeitraums wird durch erneutes kontrolliertes Aussetzen gegenüber dem Allergen das Individuum auf seine allergische Reaktion überprüft. Sollte diese Reaktion erneut auftreten, so werden die oben genannten Schritte wiederholt. D. h., das Allergen wird wieder entzogen und möglichst bald nach Entfernung des Allergens, gegebenenfalls durch zusätzliche Waschung und Kleiderwechsel, wird das Individuum innerhalb von sechs Stunden, wie innerhalb von vier Stunden, insbesondere innerhalb von zwei Stunden erneut mit der erfindungsgemäßen Allergie modulierenden Zusammensetzung behandelt.

Anschließend findet wieder eine 36- bis 92-stündige möglichst konsequente Karenzzeit statt, in dem das Individuum dem Allergen nicht ausgesetzt ist.

Sollte das Allergen im Körper vorliegen und somit ein vollständiges Entziehen nicht möglich sein, wie z. B. bei einem Insektenstich oder nach Nahrungsaufnahme, wird die erfindungsgemäße Zusammensetzung täglich verabreicht, z. B. über mindestens 3 Tage.

Diese Behandlungsschritte werden solange wiederholt - üblicherweise 3- bis 6-mal - bis das Individuum gegenüber dem Allergen nach Aussetzen nur noch geringe oder keine allergische Reaktion mehr aufzeigt. Dann wird nach Allergenentfernung innerhalb von zwei Stunden die erfindungsgemäßen Allergie modulierenden Zusammensetzung nochmals verabreicht. Anschließend sollte bevorzugt eine Karenzzeit von mindestens einer Woche eingehalten werden.

Nun erfolgt eine weitere kontrollierte Allergenexposition.
Zeigt das Individuum erneut allergische Symptome, schließen sich erneut o.g. Behandlungsschritte an: i.) Kontrollierte Allergenexposition, ii.) beim Auftreten erster deutlicher Symptome Allergenentfernung (ggf. einschließlich Reinigung), iii.) möglichst baldige (innerhalb von zwei Stunden) mukosale Behandlung mit der erfindungsgemäßen Allergie modulierenden Zusammensetzung gefolgt von möglichst konsequenter Allergenkarenz für 36 bis 96 Stunden.

Zeigt das Indivuduum nun keine allergische Reaktion mehr, kann es für dieses Allergen als "geheilt" betrachtet werden. Für derart behandelte Individuen traten für dieses Allergen keine oder nur geringe vorübergehende (1-2 Tage) und nicht behandlungsbedürftige allergische Reaktionen auf (bisheriger Beobachtungszeitraum bis sechs Jahre).

Sollten nach einer Zeit der Symptomfreiheit wieder deutliche bis starke allergische Symptome, auch mit klinisch identischer Erscheinung wie vor dieser Behandlung auftreten, so kann dieses nicht als Versagen der zuvor durchgeführten Therapie angesehen werden, sondern das Individuum ist nun einem Allergen begegnet, gegen das es ebenfalls bereits sensibilisiert ist, das jedoch in der zuvor durchgeführten Behandlungsphase nicht vorhanden bzw. eingebunden war. In diesem Falle handelt es sich um ein bisher kausal unbehandeltes Allergen. Dies kann aber in gleicher Weise wie zuvor bis zum völligen und langfristig bis vermutlich dauerhaften Verschwinden der allergischen Symptome behandelt werden.

Generell beruht dieses zuverlässig kausale Allergietherapieverfahren auf folgenden Prinzipien:
1. Es induziert eine epitopspezifische, körpereigene, langlebige Immunkontrolle, die überschießende Immunreaktionen gegen diese Epitope und damit allergische Symptome verhindert. Epitope sind die immunologische relevanten Bereiche eines Antigens bzw. Allergens, die das Immunsystem mit seinen spezifischen Rezeptoren (Antikörpern, T-Zell-Antigenrezeptoren) tatsächlich erfassen kann.
2. Es nutzt von den natürlichen Allergenen oder gezielt angebotenen Allergenpräparationen nur diejenigen aus, die für das allergische Individuum relevant sind. Daher die hohe Effizienz dieser Behandlungsform.
3. Eine Allergenexposition unmittelbar vor der Behandlung mit der erfindungsgemäßen Allergie modulierenden Zusammensetzung ist zwingend, um die epitopspezifischen kontrollierenden Immunzellen zu aktivieren.
4. Solche aktivierten, epitopspezifischen, kontrollierenden Immunzellen werden über Zellen der Schleimhaut, die von der erfindungsgemäßen Allergie modulierenden Zusammensetzung nun stimuliert werden, z.B. durch deren Botenstoffen (wie Zytokine und Chemokine) so moduliert, dass sie sich zu potenten epitopspezifischen Kontrollzellen entwickeln.
5. Diese Entwicklung erfolgt umso effizienter, je weniger diese Kontrollzellen durch Anwesenheit von Allergen (also den allergenen Epitopen) nach der Therapie hemmend beeinflusst werden. Deshalb ist eine möglichst vollständige konsequente Allergenkarenz für 36 bis 96 Stunden nach Verabreichen der erfindungsgemäßen Zusammensetzung erforderlich.
6. Die Häufigkeit der Behandlungsphasen und die Dauer dieser Allergietherapie hängen von dem individuellen qualitativen und quantitativen Bedarf an epitospezifischen Kontrollzellen ab. Maßgebend dafür sind vor allem der Grad und die Breite der individuellen Sensibilisierung, die Anzahl unterschiedlicher Epitope gegen die ein Individuum allergisch ist, die Menge der allergischen Epitope, die in einer Behandlungsphase eingebunden sind und der Grad der Abwesenheit von allergischen Epitopen während der Karenzphasen.
7. Sind für die exponierten allergischen Epitope qualitativ und quantitativ genügend spezifische Kontrollzellen induziert, so ist das Individuum durch deren Langlebigkeit längerfristig bis vermutlich dauerhaft vor allergischen Symptomen gegen diese Epitope geschützt. Dies gilt insbesondere, wenn das Individuum nach erfolgreicher Therapie (d.h bis zur vollständigen Symptomfreiheit) nachfolgend häufig mit Substanzen in Berührung kommt, die diese Epitope tragen. Durch diesen häufigen Allergenkontakt werden nun keine allergischen Symptome mehr ausgelöst, jedoch die Kontrollzellen immer wieder stimuliert, so dass sie in ausreichender Zahl und Leistungsfähigkeit vorhanden sind, um auch große Allergenmengen zu kontrollieren und zuverlässig allergische Symptome zu verhindern.
8. Bei selten auftretenden allergenen Epitopen (z. B. bei Raps, der nur für wenige Wochen im Jahr blüht), kann es sein, dass die Zahl und Aktivität der Kontrollzellen gegen längere Zeit nicht auftretende Epitope (z. B. Raps spezifische Epitope) in der mehrmonatigen Karenzzeit etwas abnimmt, so dass bei plötzlicher größerer Allergenexposition (z. B. Spaziergang oder Ausritt neben einem blühenden Rapsfeld nach fast einem Jahr Rapsallergenkarenz) nun erneut, aber meist nur schwächere allergische Symptome auftreten. Hier sind die nach längerer Zeit (ca. einem Jahr) noch vorhandenen Kontrollzellen den plötzlichen großen Allergenmengen nicht mehr ganz gewachsen. Sie werden jedoch durch diese Allergene erneut stimuliert und entwickeln sich meist schnell (innerhalb von wenigen Stunden bis Tagen) wieder zu einer Kontrollaktivität, die auch große Allergenmengen beherrschen und so allergische Symptome vermeiden kann.
9. Da saisonal und oder lokal unterschiedliche allergene Epitope auftreten und vergleichbare, meist stärkere klinische Allergiesymptome auslösen können, ist für ein oder mehrere "neue" allergene Epitope diese Therapie ebenfalls durchzuführen, bis das individuelle Immunsystem auch hierfür ausreichend Kontrollzellen mit Hilfe dieser Therapie entwickelt hat.
10. Da weder Allergene oder Allergenpräparationen noch die erfindungsgemäße Allergie modulierenden Zusammensetzung systemisch verabreicht werden, sind bisher keine negativen kurz- oder längerfristigen Nebenwirkungen beobachtet worden. Als Nebenwirkungen traten bisher nur solche auf, wie sie auch von erfolgreichen Impfungen bekannt sind: Vorübergehende (wenige Stunden währende) Erhöhung der Körperinnentemperatur bis zu 1,5 °C und etwas Müdigkeit und Abgeschlagenheit am tage nach der Behandlung. Bei intranasaler Applikation, die insbesondere bei Tieren durchgeführt wird, der erfindungsgemäßen Allergie modulierenden Zusammensetzung kann es zu kurzfristigem wässrigen Nasenausfluss kommen.

Die vorliegende Erfindung erlaubt eine spezifische Behandlung der Allergie gerichtet auf die Allergie auslösenden Epitope des exponierten Allergens, im Gegensatz zu vielen anderen Therapieansätzen, die einen unspezifischen Eingriff in das Immunsystem bewirken. Die Allergie modulierende Zusammensetzung ist dabei derart hergerichtet und ggf. konfektioniert, dass sie zur mukosalen Verabreichung bestimmt ist. Die mukosale Verabreichung beinhaltet insbesondere die orale oder intranasale Verabreichung.

Bei der Allergie modulierenden Zusammensetzung handelt es sich insbesondere um Zusammensetzungen ausgewählt aus bestimmten Stämmen oder Kulturen von Hefen, Pilzen, Bakterien, Mycoplasmen, Viren und Algen, die nach geeigenten Verfahren unter GMP-vergleichbaren Bedingungen gezüchtet, vermehrt und bearbeitet (aufgeschlossen und extrahiert) werden. Die Allergie modulierenden Zusammensetzungen sind insbesondere solche, die Pathogenassoziierte molekulare Muster (englisch: pathogen-associated molecular patterns, PAMP) oder auch MAMP's (micro-associated molecular patterns) aufweisende Strukturmotive oder Moleküle beinhalten. Diese Strukturen werden durch entsprechenden Rezeptoren (pattern recognition receptors, PRR) erkannt. Solchen Rezeptoren sind bei Immunzellen aber auch auf allen Körperzellen, so auch den Zellen der Schleimhäute, weit verbreitet. Zu diesem PRR gehören die Toll-artigen Rezeptoren, die eine wichtige Rolle bei der Auslösung einer Entzündungsreaktion spielen. Weiterhin können RAMPs (receptor activity modifying proteins oder resolution-associated molecular patterns) vorhanden sein. Dem Fachmann sind entsprechende Moleküle bekannt.

Es ist bevorzugt, dass es sich bei den Mitteln zur Behandlung der Allergie bzw. der Allergie modulierenden Zusammensetzung um ein Extrakt aus Mikroorganismen handelt. Insbesondere handelt es sich bei diesem Extrakt um einen Extrakt aus Mikroorganismen, einschließlich Pilzen, Bakterien, Mycoplasmen, Viren oder Algen. Besonders bevorzugt ist ein Hefeextrakt.

Vorliegend wird unter dem Ausdruck "Mikroorganismen" verstanden: sowohl eukaryotische als auch prokaryotische Mikororganismen einschließlich Pilzen, wie Hefen, aber auch Bakterien, Algen, Mycoplasmen und Viren.

Es ist besonders bevorzugt, dass dieser Extrakt aus Mikroorganismen einer ist erhalten durch Extraktion mit ionischen und/oder nichtionischen Tensidlösungen. Dem Fachmann sind geeignete ionische und/oder nichtionische Tensidlösungen bekannt. Hierzu gehören anionische und kationische Tensidlösungen. Es ist dabei besonders bevorzugt, dass diese Tenside anionsiche Tenside auf Basis eines Sulfates sind.

Als besonders geeignete Tensidlösungen stellten sich solche von Natriumalkylsulfat, insbesondere Natriumdodecylsulfat (SDS) heraus.

Die durch bekannte Verfahren aufgeschlossenen Mikroorganismen, wie Hefen, können z. B. solche sein, die mit ionischen Tensidlösungen, die bis zu 50 % in der Endkonzentration vorliegen können, extrahiert wurden. Diese Extrakte können dann als Lösung oder in lyophilisierter Form bereitgestellt werden. Die Extrakte in lyophilisierter Form können dann entsprechend zur mukosalen, insbesondere nasalen und oralen Verabreichung hergerichtet sein. Diese Verabreichungsformen schließen dabei insbesondere partikuläre Formen, als Aerosole aber auch in flüssiger Form, also als gelöste Zusammensetzung, ein.

In einem weiteren Aspekt richtet sich die vorliegende Anmeldung auf die Verwendung von Extrakten aus Mikroorganismen, insbesondere Extrakten aus Mikroorganismen Erhalten durch Extraktion mit ionischen und nichtionischen Tensidlösungen zur Behandlung bei Mensch und Tieren von Allergien, Autoimmunerkrankungen, Infektionserkrankungen, oder als Adjuvans in Vaccinen. Bei Pferden eignet es sich zudem zur Behandlung der Hufrehe.

Es zeigte sich, dass diese Extrakte aus Mikroorganismen, wie Hefeextrakte, in der Lage sind, die genannten Erkrankungen zu behandeln. Dabei sind die Extrakte derart hergerichtet, dass sie mukosal verabreicht werden, insbesondere oral oder intranasal. Es ist insbesondere bevorzugt, dass diese Extrakte und Zusammensetzungen nicht zur systemischen Verabreichung vorgesehen sind.

Der Extrakt ist insbesondere zur humanen oder tiermedizinischen Anwendung bei den oben genannten Erkrankungen geeignet. Es zeigte sich z. B. bei der Hufrehe, dass diese relativ schnell, wesentlich einfacher und erfolgreicher behandelt werden kann, als mit herkömmlichen Behandlungsverfahren. Eine Gabe der Extrakte bei gesunden Pferden zeigte keinerlei Nebenwirkungen und insbesondere keine Immunantwort stimulierende Wirkung auf. Bevorzugt erfolgt erfindungsgemäß eine mehrfache Anwendung des erfindungsgemäßen Extraktes. Es ist dabei bevorzugt, das Extrakt in Abhängigkeit von der Erkrankung verabreicht wird: Bei akuten Infektionserkrankungen, akuten Schüben von Autoimmunerkrankungen oder der Hufrehe wird bevorzugt an Tag 1, Tag 2, Tag 4 und an Tag 7 behandelt. Sollten die Symptome bis dahin nicht abgeklungen sein, wird entweder wöchentlich zweimal oder einmal, abhängig vom Krankheitsverlauf weiter behandelt, bis die Symptome völlig abgeklungen sind. Bei chronischen Infektions- und Autoimmunerkrankungen sowie bei der chronischen Hufrehe der Pferde wird bei schweren Erkrankungsformen alle zwei Tage eine Behandlung durchgeführt, bis sich die Symptome bessern. Dann können die Behandlungsabstände verlängert werden bis zu einer Woche. Bei weniger schweren Erkrankungsformen ist folgende Behandlungssequenz zu bevorzugen: Drei Behandlungen je im Abstand von zwei Tagen, dann eine Woche Behandlungspause, dann erneut drei Behandlungen je im Abstand von zwei Tagen. Die Dauer dieser Behandlungssequenzen sind abhängig vom individuellen Krankheitsverlauf. Dabei können die Extrakte adjuvant (d.h. zusätzlich zu anderen Behandlungsmaßnahmen, insbesondere Medikamente) oder als Monotherapie (d. h. als alleiniges Therapeutikum gegeben werden. Bei allergischen Erkrankungen ist die Therapie mit Dosen des erfindungsgemäßen Extraktes nach dem oben beschriebenen Schema in eine zwingende Sequenz von Allergenexposition, gleich anschließender Gabe einer Extraktdosis und sofort folgender Allergenkarenz für 36 bis 96 Stunden bzw. länger, eingebunden. Ohne diese Sequenz ist keine wesentliche Wirkung zu erwarten. Insbesondere ist es bevorzugt, dass der Extrakt unmittelbar bei Ausbruch der genannten Behandlung, wie Allergien, Autoimmunerkrankungen, Infektionserkrankungen oder Hufrehe verabreicht wird.

Der erfindungsgemäße Extrakt ist bevorzugt einer aus Pilzen, Bakterien, Mycoplasmen oder Viren. Insbesondere ist es bevorzugt ein Extrakt aus Hefen, wie S. cerevisiae einzusetzen.

Der Extrakt kann dabei in Form eines Lyophilisates vorliegen. Der Extrakt ist derart ausgebildet, dass er zur mukosalen Verabreichung, insbesondere intranasalen oder oralen Verabreichung geeignet ist. Dem Fachmann sind entsprechende Darreichungsformen bekannt. Der Fachmann kann weiterhin entsprechend dem zu behandelnden Individuum, ob Mensch oder Tier, entsprechend die Dosierung bemessen. Üblicherweise erfolgt dabei die Gabe des erfindungsgemäßen Extraktes in Einzeldosen nach obigen Behandlungssequenzen, in Abhängigkeit von der Erkrankung, ihres Schweregrades sowie ihres Verlaufes.

Der Extrakt kann dabei aus einem Mikroorganismus gewonnen werden, wie er natürlich vorkommt oder einem gentechnisch hergestellten Mikroorganismus, z. B. einem Mikroorganismus, der ein heterologes Molekül exprimiert. In einer bevorzugten Ausführungsform ist dieses heterolog eingeführte und exprimierte Molekül ein Zytokin, z. B. IL-2.

Bei dem erfindungsgemäßen Extrakt handelt es sich insbesondere um ein Extrakt aus Hefe, wie S. cerevisiae erhältlich durch Extraktion mit SDS. Dieser Extrakt liegt hergerichtet zur mukosalen, insbesondere nasalen oder oralen Verabreichung vor. Bevorzugt ist diese Hefe dabei keine gentechnisch veränderte Hefe.

Es konnte gezeigt werden, dass der erfindungsgemäße Extrakt nicht nur erfolgreich eine Behandlung der Hufrehe bei Pferden erlaubt, sondern auch in anderen veterinärmedizinischen und humanen Anwendungen erfolgreich sein kann.

Es zeigte sich, dass z. B. virale und bakterielle Infektionen in Pferden, Rindern, Schweinen, Hunden oder Katzen behandelt werden können. Des Weiteren konnte eine Wundheilung bei Pferden beobachtet werden. Autoimmunerkrankungen (Lupus erthematodes, Glomerulonephritis, mulitple Sklerose) und besonders Typ I-Allergien (insbesondere gegen Pollen, Insekten, Milben, Schimmelpilze, Nahrungs- bzw. Futtermittel, Medikamente, Zusatzstoffe) konnten im veterinärmedizinischen aber auch in der humanen Anwendung behandelt werden.

Die erfindungsgemäß zu behandelnden Infektionserkrankungen umfassen dabei insbesondere virale (insbesonder Herpes- und Influenzaviren), bakterielle (sowohl Gram positive wie Staphylokokken und Streptokokken und Gram negative wie E. coli) und Mycoplasmenerkrankungen (z. B. Mykoplasmen bedingte Lungenerkrankungen).

Es zeigte sich weiterhin als vorteilhaft, dass z. B. bei Allergien nach erfolgreicher Behandlung mit der erfindungsgemäßen Zusammensetzung ein regelmäßiges Aussetzen gegenüber dem Allergen die Dauer der durch die Behandlung erreichte Immunkontrolle vor allergischen Symptomen des Individuums entscheidend unterstützt. Daher ist es bevorzugt, dass die Individuen, die gegen Allergien erfindungsgemäß erfolgreich behandelt wurden, regelmäßig, z. B. einmal im Monat dem Allergen ausgesetzt werden. Dabei bleiben die Individuen symptomfrei.

Erfindungsgemäß werden unter dem Ausdruck "Individuum" Säugetiere verstanden, insbesondere Nutztiere aber auch Menschen. In einer bevorzugten Ausführungsform ist der erfindungsgemäße Extrakt insbesondere zur Behandlung von Menschen geeignet. In einer weiteren Ausführungsform ist das erfindungsgemäße Verfahren und der erfindungsgemäße Extrakt insbesondere für Tiere, insbesondere Pferde, Hunde, Katzen, große und kleine Wiederkäuer, sowie Schweine geeignet.

Der erfindungsgemäße Extrakt wird mittels Extraktion mit einer ionischen und/oder nichtionischen Tensidlösung erhalten. Dem Fachmann sind geeignete ionischen und nichtionische Tenside bekannt. Diese Tenside schließen insbesondere anionische Tenside und hier insbesondere Tenside auf Basis eines Sulfates ein. Es zeigte sich, dass Extrakte erhalten mit Natriumalkylsulfaten als Tensiden, insbesondere Natriumdodecylsulfat, SDS, zur erfindungsgemäßen Verwendung zur Behandlung von Allergien aber auch zur Behandlung von Autoimmunerkrankungen, Infektionserkrankungen, Hufrehe oder als Adjuvans in Vaccinen geeignet ist.

Das Verfahren zur Gewinnung dieser erfindungsgemäßen Extrakte schließt dabei die Kultivierung der Mikroorganismen einschließlich Pilzen, wie Hefen, Bakterien, Mycoplasmen, Viren aber auch Algen ein. Nach Anzucht und Anreicherung, z. B. durch Zentrifugation des Kulturmediums, werden die Mikroorganismen mechanisch aufgeschlossen. Dem Fachmann sind geeignete Verfahren zum mechanischen Aufschließen bekannt. Die Mikroorganismen werden anschließend mit geeigneten Tensidlösungen, insbesondere solchen die in Lebensmitteln oder pharmazeutischen Präparationen zugelassen sind, extrahiert. Der Kulturüberstand wird filtriert und anschließend lyophilisiert.

Die Tensidlösung ist z. B. in einer Konzentration von bis zu 50 Vol.-% in dem Extrakt vor Lyophilisieren vorhanden.

Schließlich werden Verfahren zur Behandlung von Allergien, Autoimmunerkrankungen, Infektionserkrankungen und Hufrehe bei Pferden bereitgestellt.

Diese Verfahren sind geeignet zur Behandlung eines Individuums, das an einer Allergie leidet. Das erfindungsgemäße Verfahren umfasst dabei die Schritte:
- Beenden des Aussetzen des Individuums gegenüber dem Allergen;
- Mukosale Verabreichung einer Allergie modulierenden Zusammensetzung wie einem Extrakt aus Mikroorganismen, insbesondere ein Extrakt aus Mikroorganismen erhaltend durch Extraktion mit ionischen und/oder nicht ionischen Tensidlösungen innerhalb von sechs Stunden, bevorzugt innerhalb innerhalb von zwei Stunden nach Beenden des Aussetzens des Individuums gegenüber dem Allergen.

Bevorzugt umfasst dieses Verfahren in einem weiteren Schritt das Durchführen einer Karenzzeit von mindestens 36 Stunden und maximal 96 Stunden, bevor das Individuum dem Allergen erneut ausgesetzt werden kann.

Bevorzugt wird die allergiemodulierende Zusammensetzung, wie der Extrakt aus Mikroorganismen, insbesondere der Extrakt aus Mikroorganismen, erhalten durch Extraktion mit ionischen und/oder nicht ionischen Tensidlösungen wiederholt verabreicht, bis die Allergiesymptome durch das exponierende Allergen nicht mehr ausgelöst werden können.

In einer Ausführungsform wird bei dem erfindungsgemäßen Verfahren dabei die Zusammensetzung täglich wiederholt verabreicht, insbesondere dann, wenn das Allergen im Körper vorliegt, zum Beispiel bei einem Insektenstich oder nach Nahrungsaufnahme.

Weiterhin wird ein Verfahren zur Behandlung eines Individuums bereitgestellt, das an einer Allergie, Autoimmunerkrankung, Infektionserkrankung oder Hufrehe bei einem Pferd leidet, das Verfahren umfasst die Schritte des Verabreichens eines Extraktes aus Mikroorganismen, insbesondere einem Extrakt aus Mikroorganismen, erhalten durch Extraktion mit ionischen und nicht ionischen Tensidlösungen, insbesondere Hefeextrakten, wobei die Verabreichung bevorzugt mukosal erfolgt.

Es ist bevorzugt, dass die Verabreichung wiederholt durchgeführt wird, insbesondere bei einer Autoimmunerkrankung, mehrfach in einem Zeitraum von 24 bis 48 Stunden, bei einer Infektion täglich über mindestens drei Tage oder bei der Hufrehe mehrfach über einen Zeitraum von einer Woche, wie an den Tagen 1, 2, 4 und 7.

Das erfindungsgemäße Verfahren ist insbesondere geeignet zur Behandlung von Säugetieren einschließlich Tier und Mensch, besonders bevorzugt für Tiere.

Eine erfolgreiche Behandlung von Allergien am Beispiel eines Pferdes (A) sowie von Hufrehe am Beispiel eines Pferdes (B) ist im Folgenden exemplarisch beschrieben:

### A) Ein Pferd mit allergischer Urticaria (Nesselsucht)

Ein Reitpferd zeigte starke und juckende Hautschwellungen in sehr unregelmäßigen Abständen und unabhängig von der Jahreszeit. Nach eingehender in vitro und Expositionsdiagnostik zeigte sich als Ursache der Hauterkrankung eine Allergie sowohl gegen Hafer, Karotten als auch Äpfel. Wenige Stunden nachdem das Tier eines dieser Futtermittel auch nur in kleinen Mengen (eine Hand voll Hafer, eine Karotte oder einen Apfel) zu sich genommen hatte, war es übersät von Hautschwellungen (Urticariae).

Daraufhin wurde diese Tier im Rahmen eines genehmigten Tierversuches mit einer erfindungsgemäßen Zusammensetzung (hier als **Substanz** bezeichnet) als Monotherapie folgendermaßen behandelt:
1. **Kontrollierte Exposition:** Dem Tier wurde eine halbe Hand voll Hafer zum Fressen geben. Daraufhin entwickelte es milde aber deutliche Urticaria-Symptome.
2. **Therapie:** Sofort wurde ein in lyophilisierter Form lange haltbare Substanz (von dieser Charge 160 mg) in 3 mL sterilem Injektionwasser gelöst und davon mittels Spritze und stumpfer Sprühkanüle je 1,5 mL in alle drei Kammern der linken und der rechten Nasengänge versprüht.
3. **Karenz:** Anschließend wurden von dem Tier für 48 Stunden jegliche Form von Hafer, aber auch Karotte oder Äpfel fern gehalten. In dieser Zeit verschwanden die Urticaria-Symptome weitgehend.
4. **Nach 48 Stunden** erfolgte eine erneute Exposition mit einer halben Hand voll Hafer. Kurz darauf entwickelte das Tier wieder deutliche Urticaria und wurde wie unter 2. und 3. beschrieben behandelt und in strikter Karenz gehalten.
5. **Nach weiteren 48 Stunden** konnten mit einer halben Hand voll Hafer nur verzögert und sehr abgeschwächt Urticaria-Symptome ausgelöst werden. Daraufhin erfolgte eine dritte Behandlung wie unter 2. beschrieben. Die nun anschließende Karenz (kein Hafer, keine Karotten und keine Äpfel) wurde nun für 1 Woche eingehalten.
6. **Erste Prüfexposition:** Anschließend erhielt das Tier erst eine volle Hand mit Hafer, zeigte jedoch keinerlei Urticaria-Reaktion. Ohne Behandlung mit der Substanz blieb das Tier für weitere 24 Stunden in Allergenkarenz (kein Hafer, keine Karotten, keine Äpfel).
7. **Zweite Prüfexposition:** Nach diesen 24 Stunden erhielt das Tier nun seine normale Haferration (3 kg). Als es daraufhin auch keinerlei Urticaria-Symptomatik zeigte, wurde es als "geheilt" von seiner Haferallergie angesehen. Seither (in den bisherigen vier Jahren) hat es auf Hafer nie wieder eine allergische Reaktion gezeigt, obwohl es fast täglich Hafer erhält.
8. **Tolerant gegenüber Hafer** wurde dem Tier nun **eine Karotte** verabreicht. Innerhalb kurzer Zeit entwickelte es eine starke Urticaria. Somit war zwar gegen allergische Epitope im Hafer eine ausreichend wirkungsvolle epitopspezifische Immunkontolle erreicht worden, nicht jedoch gegen Karotten charakteristische und für dieses Tier allergische Karottenepitope, da diese bei der bisherigen Behandlung nicht exponiert worden waren.
9. Nun wurde entsprechend der Punkte 2. bis. 7. Verfahren mit der Ausnahme, dass in der Karenz nun Hafer, jedoch weder Karotten noch Äpfel gegeben wurden., Bis zum Ende dieser Behandlung (und die ganz nachfolgende Beobachtungszeit von ca. 4 Jahren) das Tier nun auch eine stabile Karotten-Epitop spezifische Immunkontrolle entwickelt hatte, Diese erlaubt ihm seither, jede Menge an Karotten zu fressen, ohne eine allergische Reaktion.
10.Trotz Toleranz gegen Hafer und Karotten löste ein einziger Apfel erneut deutliche Urticaria-Symptome aus. Die Apfel charakteristischen Epitope, gegen das diese Pferd auch allergisch war, stellten nun neue allergene Epitope dar, die bei den bisherigen Behandlungen nicht beteiligt waren. Deshalb erneut die volle Urticaria-Symptomatik wie zu Beginn vor der Behandlung.
11.Wurde das Tier nun entsprechend den Punkten 2. bis 7. Erneut mit der Substanz unmittelbar nach Apfelexposition behandelt und einer anschließenden Apfelkarenz (bei Verabreichung von Hafer und Karotten) unterzogen, war nach ebenfalls drei Behandlungen mit der Substanz auch eine Toleranz gegen Apfelantigene erreicht. Dies erlaubt dem Tier seither problemlos Äpfel zu verzehren.
12.Bei der Verabreichung einzelner anderer Apfelsorten als der zur Exposition vor der Therapie eingesetzten Sorte traten wieder leichte Urticaria-Symptome auf. Diese verschwanden teils nach wiederholter Gabe dieser anderen Apfelsorte von alleine. Bei etwas stärkeren Symptomen, die länger als zwei Tage anhielten, wurden diese "neuen" Epitope der anderen Apfelsorte meist mit einer weiteren Subtanzbehandlung gemäß den Punkten 2. und 3. Ebenfalls und bisher anhaltend unter Immunkontrolle gebracht.

### B) Ein American Quarter Horse mit chronisch rezidivierender Hufrehe

Dieses Tier litt seit mehreren Jahren an Hufrehe mit drei bis 4 Schüben pro Jahr. Alle gängigen und meist langwierigen Therapieverfahren waren bereits angewandt worden und hatten lediglich zu etwas verlängerten Schubintervallen geführt. Die vielen Kosten und die geringe Reitbarkeit veranlassten den Besitzer, das Tier zu verschenken. Der neue Besitzer nahm über seinen Tierarzt an einem genehmigten Tierversuch teil, der den Einsatz eines erfindungsgemäßen Extraktes aus Mikroorganismen mit ionischen Tensiden (hier als "Extrakt" bezeichnet) erlaubte. Dieser Extrakt wurde nach orthopädischer Hufkorrektur als Monotherapie in folgender Weise eingesetzt:
1. **Therapie:** 150 mg des lyophilisierten Extraktes wurden in 3 mL sterilem Injektionswasser gelöst und dem Tier davon je 1,5 mL in die 3 Gänge der linken und der rechten Nase mittels stumpfer Sprühkanüle versprüht.
2. **Bis zum nächsten Tag** hatte sich die Rehesymptomatik sogar deutlich verschlechtert, so dass das Tier lieber lag als Stand. Dennoch wurde die Behandlung ca. 24 Stunden nach der ersten Behandlung wie unter 1. wiederholt.
3. **Als der Tierarzt nach weiteren 48 Stunden** zu diesem Pferd kam, leif es ihm bereits aus der box entgegen. Die Symptomatik hatte sich erheblich verbessert, war aber noch sehr deutlich vorhanden. Es erfolgte eine dritte

Behandlung wie unter 1.
4. **Nach weiteren drei Tagen, also an Tag 7** nach der Erstbehandlung hatte sich der Rehezustand weiter stark verbessert, jedoch keineswegs vollständig. Nun erhielt das Pferd seine vierte Behandlung wie unter 1.
5. Unter zunehmender Besserung des Laufverhaltens wurde nun noch dreimal im **Abstand von je 1 Woche** wie unter 1. behandelt.
6. Danach (somit insgesamt sieben Behandlungen mit dem Extrakt als Monotherapie) lief das Pferd einwandfrei, so dass es langsam wieder geritten werden konnte.
7. Inzwischen sind fünf Jahre vergangen, in denen das Pferd in den letzten vier Jahren zum Westernreiten eingesetzt wird. ein Reherezidiv ist seither noch nicht wieder aufgetreten.

### Allergische Erkrankung bei der Katze:

### Patient: Kater "Carlos"

### Anamnese:

Im Alter von 5 Jahren: Einlieferung ins Tierheim mit Poly-Allergie, insbes. gegen ein breites Spektrum an Nahrungsmitteln. Daraufhin Depotcorticosteroide (CS) und Futterumstellung: Unter CS symptomfrei, bei Abklingen der CS-Wirkung sofort wieder Juckreiz, Nießen. Akne und Alopezie (bes. an Hals und Gesicht), gerötete Augen sowie zunehmende "Unsauberkeit".

Nach einem Jahr CS-Behandlung lässt die CS-Wirkung deutlich nach, es werden immer höhere Dosen benötigt und es wird dennoch keine vollständige Symptomfreiheit mehr erreicht. Zusätzliche Gabe von Antibiotika und Homöopathika sind wirkungslos. Lediglich eine Kombination aus Depot-CS und Gestagenen kann den Zustand noch erträglich halten.

Im Alter von 8 Jahren verschlechtert sich sein Zustand selbst unter CS & Gestagen derart (er hatte inzwischen auch eine Cushing-Symptomatik entwickelt), dass eine Euthanasie erwogen wird.

### Therapie:

Da kein Futter bekannt war, auf das "Carlos" nicht allergisch reagierte, und die Symptomatik trotz CS- und Gestagengabe sehr ausgeprägt war, suchte man zuerst nach einem - für "Carlos" minimal allergenen Futte, während sehr langsam die CS- und Gestagenbehandlung ausgeschlichen wurde.

Anschließend, im Spätsommer, wurde mit der Gabe eines erfindungsgemäßen Hefeextrakts (im Folgenden TEM bezeichnet) als Monotherapie begonnen: Jeden 2. Tag TEM in 3 mL aqua dest. oral. Nach der 3. Gabe verbesserte sich der Zustand deutlich: Weniger Juckreiz, weniger Unruhe, verbesserte Reinlichkeit. Danach trat ein Stillstand auf, also keine weiter klinische Besserung, da keine temporäre Allergenfreiheit geschaffen werden konnte. Die 2-tägliche Dosis wurde auf 4 mL erhöht, was - trotz permanenter Anwesenheit von Allergen im Futter zu einer langsamen aber stetigen Minderung der Symptome führte.

Im Januar des 9. Lebensjahres benötigte "Carlos" seine letzten TEM-Gaben, da inzwischen alle Allergiesymptome verschwunden waren und inzwischen unterschiedliche, für "Carlos" allergenen Futtermittel verabreicht worden waren.

Nach Ende der TEM-Monotherapie - hier über 6 Monate - vertrug "Carlos" alle ihm angebotenen Futtermittel problemlos und zeigte bis zu seinem Tod im 12. Lebensjahr kein Zeichen einer Allergie mehr. Lediglich die "verminderte Sauberkeit" behielt er bis zu seinem Lebensende bei.

Trotz der langen Behandlung mit TEM oral wurden zu keiner Zeit unerwünschte Nebenwirkungen beobachtet.

### Allergische Erkrankung beim Hund:

### Patient "Amie"

### Anamnese:

5 jährige Terrierhünden mit progressiven, zunehmend corticosteroidresistenten polyallergischen Haut- und Schleimhautsymptomen seit mehreren Jahren und immer weniger therapierbar. Besteht seit mehreren Jahren: V. a. Allergie gegen Futtermittel und Hausstaubmilben, deshalb nahezu ganzjährig mit Schwankungen. Unter systemischer plus topischer Corticosteroid (CS)-Gabe anfangs symptomatische Besserung, die jedoch immer kürzer und schwächer ausfiel. Zudem werden CS-Nebenwirkungen (Cushing, verdünnte Haut) deutlich.

### Therapie:

Die bakterielle Hautentzündung wurde durch Cefovecin-Gabe (Convenia®) kontolliert und die Hausstaubexposition durch eine Wohnungswechsel weitgehend reduziert.

Nun konnte die Allergie gegen verschiedene Futtermittel mit Hilfe von ca. 10 oralen Gaben von TEM so unter Immunkontrolle gebracht werden, dass alle bis dahin unverträglichen Futtermittel nun symptomfrei vertragen wurden. Dieses auch, nachdem das Tier wieder "ihren" Hausstaubmilben ausgesetzt wurde. Da diese Exposition nun gezielt durchgeführt und von Karenztagen abgelöst wurde, war mit 6 TEM-Gaben auch gegen diese Milben eine symptomfreie "Toleranz" erzielt worden. Nun wurde auch das Antibiotikum (Cefovecin) abgesetzt. Die daraufhin wiederkehrende Hautentzündung mit starkem Juckreiz konnte durch 12 weitere TEM-Gaben vollständig unter Kontrolle gebracht werden. Der allergiefreie Zustand ohne Hautprobleme hielt für mindestens 2 Jahre ohne erneute TEM-Gabe oder eine andere Therapie an. In der Behandlungszeit und in der Beobachtungszeit danach war keinerlei unerwünschte Nebenwirkung durch die TEM-Gabe festzustellen.

### Allergische Erkrankungen beim Pferd:

### Patient mit Erythema multiforme

### Anamnese:

"Sandorett", Warmblutwallach und erfolgreiches Turnierpferd zeigt ab seinem 5. Lebendjahr (Lj) insbesondere vom Frühjahr bis Herbst, in geringerer Häufigkeit auch im Winter rezidivierende und konfluierende Erythemae multiforme, die nicht auf Antihistaminica ansprechen. Mit Dexamethason sind die Symptome zwar kurzfristig zu beheben, kehren nach Reduktion oder Absetzen sehr bald wieder. Bis zu seinem 8. Lj. werden die Schübe häufiger und in der Ausbreitung und Intensität so stark, dass das Pferd kaum noch reitbar ist und schon lange an keinem turnier mehr teilnehmen konnte. Zudem lehnte der Besitzer weitere Corticosteroid (Cs)behandlungen ab, um dem Tier daraus zu erwartende Nebenwirkungen zu ersparen.

### Therapie:

Im 8. Lj. erfolgte nach vollständigem Ausschleichen der bisherigen Dexamethason-Behandlung eine Monotherapie mit TEM: "Sandorett" erhielt das TEM gelöst in aq. dest. in Volumina von je 3 mL intranasal versprüht. Nach 3 Applikationen im Abstand von je 2 Tagen war bereits eine deutliche Reduktion der durch das Absetzen der Cs-Behandlung wieder stark aufgetretenen Erythema multiforme-Stellen zu verzeichnen. Im Anschluss an 3 weitere intranasale TEM-Gaben verschwanden die Symptome vollständig, so dass das Pferd nach 4 wöchiger TEM-Behandlung wieder reitbar wurde. Von Mitte Februar bis Ende September blieb "Sandorett" rezidivfrei. Ein schwaches Erythema-Rezidiv Ende Sept. war mit 3 intranasalen TEM-Behandlung wieder vollständig zu beheben. In den beiden folgenden Jahren konnte Sandorett wieder intensiv auftrainiert werden und Turniere besuchen. Zu keiner Zeit während und nach der TEM-behandlung waren unerwünschte Nebenwirkungen zu beobachten.

### Patient mit allergischem "Headshaking" (HS), entsprechend der allergischen Rhinitis des Menschen ("Heuschnupfen")

### Anamnese:

"Filou", ein Hannoveraner-Wallach und ausgebildetes Military-Pferd entwickelt im 5. Lebensjahr (Lj.) vom zeitigen Frühjahr bis Herbst ein allergisches Headshaking (HS). Dies wird im Sommer so heftig, dass das Pferd bei der Arbeit sehr unaufmerksam und wegen heftiger "spontaner" Kopfreaktionen oft nicht mehr reitbar ist. Teilnahme an Turnieren war damit ganz ausgeschlossen. Alle Schutz- und Behandlungsmaßnahmen waren nur von kurzfristigem Erfolg, wenn überhaupt.

### Therapie:

Im 8. Lj. Mitte Mai begann die Monotherapie mit intranasaler Applikation von TEM. Nach der 3. TEM-Gabe war "Filou" bereits deutlich ruhiger om Freien und zeigte sogar beim Longieren erkennbar schwächere HS-Symptome. Nach 3 weiteren TEM-Gaben zeigt "Filou" in Ruhe und leichter Bewegung weder im Stall noch im Freien oder auf der Weide Anzeichen von HS. Lediglich bei intensiverem Longieren traten noch leichte HS-Symptome auf. 2 weitere TEM-Behandlungen führten zum völligen Verschwinden jeglicher HS-Symptome, auch unter intensiven Bewegungsbelastungen. Ab Ende Juni konnte wieder die regelmäßige Arbeit mit "Filou" beginnen, Er zeigte seither keine HS-Symptome mehr und konnte in seinem 9. Lj. wieder an den Military Meisterschaften teilnehmen, was durch sein HS in den letzten 4 Jahren nicht möglich war. Unter und nach der TEM-Behandlung wurden keinerlei unerwünschte Nebenwirkungen gesehen.

### Patient mit allergischer chronisch obstruktiver Bronchitis (COB)

### Anamnese:

"Chester", ein Brandenburger Wallach, leidet seit Jahren an einer chronisch obstuktiven Bronchitis (COB) mit leicht auslösbarem Husten, insbesondere wenn er auf Stroh steht oder mit trockenem Heu gefüttert wird. Im funktionellen Allergietest (FIT) zeigte er eine deutliche funktionelle Sensibilisierung gegen Staub- und Futtermilben. Eine reiterliche Arbeit war mit ihm seit Jahren schon nicht mehr möglich. Auf frische Späne gestellt und mit gründlich gewaschenem Heu gefüttert, reduzierte die COB-Symptomatik weitgehend. Dies war durch die Nachbarstallungen nicht ausreichend sicher durchführbar, so dass es immer wieder zu allergischen Schüben kam.

### Therapie:

Im 22. Lj. von "Chester" wurde eine intranasale TEM-Monotherapie durchgeführt: Unter weitgehend allergenfreien Bedingungen (Späne als Einstreu und gewaschenes heu) wurde nahezu Symptomfreiheit erreicht. Nun wurde das Tier kontrolliert mit trockenem Heu exponiert, bis es deutlichen Hustenreiz zeigte. Anschließend wurde mit TEM beidseitig intranasal behandelt und eine Karenzphase angeschlossen. Dieser Ablauf wurde insgesamt 4-mal wiederholt. Nach der 2. Behandlung zeigte sich bereits eine leicht verminderte Reaktion auf die Heuexposition. Nach der 4. Behandlung konnte mit trockenem Heu keinerlei Hustenreaktion oder verschärfte Atmung mehr ausgelöst werden. Seitdem wird "Chester" mit trockenem Heu gefüttert und kann auf Stroh stehen, ohne erkennbare Atembeschwerden oder gar Husten zu zeigen. Die Beobachtungszeit ist 2 Jahre. Während und nach der TEM-Behandlung gab es keinerlei unerwünschte Nebenwirkungen.

### Arzneimittelallergie

### Anamnese:

Der 16 j. russischer Vollblutwallach "Monaco" zeigte bei einer Augenbehandlung heftige allergische Reaktionen nach Gabe von Neomycin-Augensalbe: Anschwellen der Konjunktiven bis hin zum Verschluss des Auges, starkes Jucken mit Tränenfluss und periokuläre Alopezie. Diese Symptome klangen nur langsam die über mehrere Tage ab und beunruhigten das Pferd sehr.

### Therapie:

Nach vollständigem Abklingen der vorausgegangenen Symptome wurde am linken Auge ca. 3 mm (therapeutische Dosis ist 15 bis 20 mm) Neomycin-Salbenstrang auf die Conjunctiva aufgebracht. Innerhalb weniger Minuten schwoll die Conjunctiva stark an, tränte und es entwickelte sich ein massiver Juckreiz in diesem Auge. Umgehend wurde das Tier beidseitig intranasal mit TEM behandelt. Innerhalb von 30 bis 60 Min. war ein erkennbarer Rückgang der Symptome (Schwellung, Tränenfluss, Juckreiz, Unruhe) zu verzeichnen.

Vollständig waren alle Symptome jedoch erst nach ca. 36 h verschwunden.

72 h nach der ersten Exposition im linken Auge wurde im rechten Augen ebenfalls ca. 3 mm Neomycin-Augensalbe aufgebracht und auf die Reaktion gewartet. Sie blieb jedoch vollständig aus, so dass eine 2. TEM-Behandlung unterblieb. 24 h später wurde nun wieder das linke Auge mit nochmals 3 mm der Salbe exponiert. Jedoch auch hier blieb nun jedes Anzeichen einer Reaktion aus. Daraufhin wurde am nächsten Tag in das rechte Auge die volle therapeutische Dosis an Neomycin-Augensalbe eingebracht. Als auch hier und ebenso am darauffolgenden Tag im linken Auge die volle Salbendosis ohne eine erkennbare Reaktion blieb, wurde eine Toleranz gegenüber dem oder den Allergenen in der Neomycin-Augensalbe postuliert. Somit hat eine einzige TEM-Behandlung zu dieser Toleranzinduktion geführt. Seither (Beobachtungszeit = 5 Jahre) hat "Monaco" nie wieder auf Neomycin allergisch reagiert.

### Autoimmunerkrankung beim Pferd:

### Rezidivierende equine Uveitis (ERU).

### Anamnese:

Der 13 jährige PRE (Pura Raza Espanola)-Wallach "Travieso" leidet seit mehreren Jahren an einer rechtsseitigen chronisch rezidivierenden Uveitis mit 3-4 Schüben pro Jahr. Auf die klassische symptomatische Behandlung mit Atropin, Nicht-steroidalen Antiphlogistica und antibiotischer sowie steroidaler Augensalbe reagierte das Tier nur noch mit geringer temporärer Abschwächung der Symptome. Auf Atropin reagierte es gar nicht mehr.

Da sich zunehmend die Erkenntnis durchsetzt, dass es sich bei der ERU hauptsächlich um eine Autoaggressionserkrankung handelt, wurde eine Behandlung mit TEM vorgenommen.

### Therapie:

Ende Januar erleidet "Travieso" ein massives Rezidiv: Auge eingefallen, drittes Augenlid sehr weit vorgefallen, Iris vorgefallen, extrem schmerzhaft , wenn Auge auch nur am meist geschlossenen Lid berührt wird und starker Juckreiz, dadurch leicht Fluorescein-positive Cornealäsion,. Conjunctiven so stark geschwollen, dass sie schon allein das Auge bei offenem Lid bedeckt haben. Daraufhin beidseitige intranasale Behandlung mit TEM plus Augensalbe mit Vit. A und Gentamycin wegen der Cornealäsion.

Bereits nach 24 h deutliche Besserung: Abschwellende Conjunctiven, leicht geöffnetes Auge, weniger Tränenfluss, weniger Schmerzen (das Tier erlaubt nun die Berührung der Augenlider und die Salbeneingabe). 48h nach erster TEM-Gabe erneute TEM-Behandlung mit eindrucksvoller Besserungstendenz und deutlich lebhafteres verhalten von "Travieso". Nach einer 3. TEM-Gabe weitere deutliche Besserung des Auges, fast abgeschwollene Conjunctiven, reponiertes drittes Augenlid und weitgehend physiologische Augengröße. § Wochen nach Behandlungsbeginn ist das Auge schmerzfrei, ohne Entzündungszeichen und mit klarem Glaskörper ohne Schlieren oder Fibrinfäden.

Im Gegensatz zu früher blieb "Travieso" bis Dezember ohne Rezidiv. Ein schwächeres Rezidiv im Dezember konnte mit 2 TEM-Behandlungen innerhalb von 3 Tagen wieder behoben werden. Im anschließenden Jahr hat "Travieso" kein weiteres Rezidiv. Auch waren zu keiner Zeit unerwünschte Nebenwirkungen durch die Gabe von TEM zu beobachten.

### Viruserkrankung beim Pferd:

### Herpesvirusinfektion

### Anamnese:

Die Quarterhorse-Stute "Beauty flower" leidet seit ihrem 4. Lebensjahr (Lj.) an chronischem ganzjährigen therapieresistenten Husten, der weder durch Futter-, Einstreu- oder Stallwechsel bzw. Weidegang zu beeinflussen ist. Nur bei Bewegungsbelastung wird er deutlich stärker. Corticosteroidbehandlungen bringen nur minimale temporäre Abschwächung des Hustens. Auskultatorisch und bronchoskopisch ist keine Bronchenbeteiligung festzustellen. Ursächlich wurden multiple Pusteln und Papeln im Laryngopharynx bedingt durch Herpesviren ausgemacht.

### Therapie:

Im 7. Lj. wurde eine intranasale Monotherapie mit TEM vorgenommen: Nach der ersten Behandlung war der Husten im Ruhezustand für 1 Tag deutlich verstärkt und von heftigem Speichelfluss und Futterverweigerung begleitet. Dennoch wurde nach 24 h die nächste TEM-Gabe borgenommen. Darauf besserte sich der Husten erheblich, der Speichelfluss normalisierte sich und es wurde wieder etwas Futter aufgenommen. Nach der 3. TEM-Behandlung hustete "Beauty flower" nur noch gelegentlich und deutlich schwächer. Speichelfluss und Futteraufnahme waren wieder normal. Ohne weitere TEM-Behandlung besserte sich der Husten täglich, bis er 2 Wochen nach der letzten TEM-Gabe weder mechanisch noch durch starke Laufbelastung auszulösen war. Bronchoskopisch waren im Laryngopharynx weder Papel noch Pusteln oder andere Entzündungszeichen zu sehen. In der nachfolgenden Beobachtungszeit von einem Jahr traten weder ein Rezidiv noch unerwünschte Nebenwirkungen auf.

## Patentansprüche

1. Hefeextrakt erhalten durch Extraktion mit ionischen und nichtionischen Tensidlösungen zur Verwendung bei der Behandlung von Allergien, wobei der Extrakt mukosal verabreicht wird und die Verabreichung innerhalb von sechs Stunden, bevorzugt innerhalb von zwei Stunden, nach Ende des Aussetzens gegenüber dem Allergen erfolgt.

2. Hefeextrakt zur Verwendung nach Anspruch 1, wobei nach Verabreichung des Hefeextraktes eine Karenzzeit von mindestens 36 Stunden und maximal 96 Stunden eingelegt wird, bevor das Individuum dem Allergen erneut ausgesetzt werden soll.

3. Hefeextrakt zur Verwendung nach Anspruch 1 oder 2, wobei der Hefeextrakt nach erneutem Aussetzen und Entzug des Allergens innerhalb von maximal zwei Stunden verabreicht wird.

4. Hefeextrakt zur Verwendung nach einem der vorherigen Ansprüche, wobei dieser wiederholt verabreicht wird, bis die Allergiesymptome durch das exponierende Allergen nicht mehr ausgelöst werden können.

5. Hefextrakt zur Verwendung nach einem der vorherigen Ansprüche, wobei das Individuum nach einer Behandlung mindestens eine Woche dem Allergen nicht ausgesetzt ist.

6. Hefeextrakt zur Verwendung nach einem der vorherigen Ansprüche, **dadurch gekennzeichnet, dass** dieser durch Extraktion mit ionischem Tensid, insbesondere anionischem Tensid, insbesondere einem Tensid auf Basis eines Sulfates erhalten wird, wie Natriumalkylsulfat, insbesondere Natriumdodecylsulfat.

7. Hefeextrakt zur Verwendung nach einem der vorherigen Ansprüche, wobei dieser einer aus SDS-extrahierten Hefen ist.

8. Hefeextrakt zur Verwendung nach einem der vorherigen Ansprüche, wobei dieser geeignet ist zur Verwendung bei der Behandlung von Allergien in Individuen, die keine Kortisonbehandlung erhalten.

9. Hefeextrakt zur Verwendung nach einem der vorherigen Ansprüche, wobei dieser als Lyophilisat vorliegt zur mukosalen Verabreichung, insbesondere intranasalen oder oralen Verabreichung.

10. Hefeextrakt zur Verwendung nach einem der vorherigen Ansprüche, **dadurch gekennzeichnet, dass** die Hefen mindestens ein heterologes Molekül exprimieren, insbesondere ein Zytokin.

11. Hefeextrakt zur Verwendung nach einem der vorherigen Ansprüche 1 bis 9, wobei der Extrakt einer ist erhalten durch Extraktion mit SDS, dieser Extrakt in lyophilisierter Form vorliegt, zur mukosalen, insbesondere nasalen und oralen Verabreichung und die Hefen keine gentechnisch veränderten Hefen sind.

12. Hefeextrakt zur Verwendung nach einem der Ansprüche 1 bis 11 zur Behandlung eines Individuums, das an einer Allergie leidet, derart, dass das Aussetzen des Individuums gegenüber den Allergen beendet wird, der Hefeextrakt gemäß einem der Ansprüche 1 bis 11 mukosal innerhalb von sechs Stunden, bevorzugt innerhalb von zwei Stunden, nach Beenden des Aussetzens des Individuums gegenüber den Allergen verabreicht wird.

## Claims

1. Yeast extract obtained by extraction using ionic and non-ionic surfactant solutions for use in the treatment of allergies, wherein the extract is administered mucosally and the administration takes place within six hours, preferably within two hours, after the end of the exposure to the allergen.

2. Yeast extract for use according to Claim 1, wherein an abstinence time of at least 36 hours and not more than 96 hours is observed after administration of the yeast extract before the individual is to be re-exposed to the allergen.

3. Yeast extract for use according to Claim 1 or 2, wherein the yeast extract is administered within not more than two hours after renewed exposure and withdrawal of the allergen.

4. Yeast extract for use according to any of the preceding claims, wherein said extract is administered repeatedly until the allergy symptoms can no longer be triggered by the exposing allergen.

5. Yeast extract for use according to any of the preceding claims, wherein the individual is not exposed to the allergen for at least one week after a treatment.

6. Yeast extract for use according to any of the preceding claims, **characterized in that** said extract is obtained by extraction using an ionic surfactant, especially an anionic surfactant, especially a surfactant based on a sulfate, such as sodium alkyl sulfate, especially sodium dodecyl sulfate.

7. Yeast extract for use according to any of the preceding claims, wherein said extract is one from SDS-extracted yeasts.

8. Yeast extract for use according to any of the preceding claims, wherein said extract is suitable for use in the treatment of allergies in individuals who are not obtaining cortisone treatment.

9. Yeast extract for use according to any of the preceding claims, wherein said extract is present as a lyophilisate for mucosal administration, especially intranasal or oral administration.

10. Yeast extract for use according to any of the preceding claims, **characterized in that** the yeasts express at least one heterologous molecule, especially a cytokine.

11. Yeast extract for use according to any of the preceding Claims 1 to 9, wherein the extract is one obtained by extraction using SDS, said extract is present in lyophilized form for mucosal, especially nasal and oral, administration and the yeasts are not genetically modified yeasts.

12. Yeast extract for use according to any of Claims 1 to 11 for treating an individual suffering from an allergy such that the exposure of the individual to the allergen is ended and the yeast extract according to any of Claims 1 to 11 is administered mucosally within six hours, preferably within two hours, after the end of the exposure of the individual to the allergen.

## Revendications

1. Extrait de levure obtenu par extraction avec des solutions de tensioactifs ioniques et non ioniques et destiné à être utilisé dans le traitement d'allergies, l'extrait étant administré par voie muqueuse et l'administration étant effectuée dans les six heures, de préférence dans les deux heures, suivant la fin de l'exposition à l'allergène.

2. Extrait de levure destiné à être utilisé selon la revendication 1, une période d'attente d'au moins 36 heures et de 96 heures maximum étant observée après l'administration de l'extrait de levure avant que l'individu ne soit à nouveau exposé à l'allergène.

3. Extrait de levure destiné à être utilisé selon la revendication 1 ou 2, l'extrait de levure étant administré dans un délai maximum de deux heures après une nouvelle exposition à l'allergène et l'élimination de celui-ci.

4. Extrait de levure destiné à être utilisé selon l'une des revendications précédentes, l'administration étant effectuée de manière répétée jusqu'à ce que les symptômes d'allergie ne puissent plus être déclenchés par l'allergène exposé.

5. Extrait de levure destiné à être utilisé selon l'une des revendications précédentes, l'individu n'étant pas exposé à l'allergène pendant au moins une semaine après le traitement.

6. Extrait de levure destiné à être utilisé selon l'une des revendications précédentes, **caractérisé en ce que** celui-ci est obtenu par extraction de tensioactif ionique, notamment un tensioactif anionique, notamment un tensioactif à base de sulfate, tel que l'alkylsulfate de sodium, notamment le dodécylsulfate de sodium.

7. Extrait de levure destiné à être utilisé selon l'une des revendications précédentes, celui-ci étant un extrait de levure SDS.

8. Extrait de levure destiné à être utilisé selon l'une des revendications précédentes, celui-ci étant approprié pour être utilisé dans le traitement d'allergies chez des individus qui ne reçoivent pas de traitement à la cortisone.

9. Extrait de levure destiné à être utilisé selon l'une des revendications précédentes, celui-ci se présentant sous forme de lyophilisat destiné à une administration par voie muqueuse, en particulier une administration par voie intranasale ou orale.

10. Extrait de levure destiné à être utilisé selon l'une des revendications précédentes, **caractérisé en ce que** les levures expriment au moins une molécule hétérologue, notamment une cytokine.

11. Extrait de levure destiné à être utilisé selon l'une des revendications précédentes 1 à 9, l'extrait étant un extrait obtenu par extraction avec du SDS, cet extrait étant sous forme lyophilisée pour une administration par voie muqueuse, notamment nasale et orale et les levures n'étant pas des levures génétiquement modifiées.

12. Extrait de levure destiné à être utilisé selon l'une des revendications 1 à 11 pour le traitement d'un individu souffrant d'une allergie de manière à cesser l'exposition de l'individu à l'allergène, l'extrait de levure selon l'une des revendications 1 à 11 étant administré par voie muqueuse dans les six heures, de préférence dans les deux heures suivant la fin de l'exposition de l'individu à l'allergène.
